# EUROPEAN PATENT APPLICATION

(11) **EP 4 309 703 A1**
(43) Date of publication of application: **24.01.2024**
(21) Application number: 22186255.0
(22) Date of filing: 21.07.2022
(51) Int. Cl.: A61M 5/20, A61M 5/315

(54) **AN INJECTION DEVICE COMPRISING A RELEASABLE SNAP FIT CONNECTION FOR A CLOSURE CAP**

(71) Applicant: Ypsomed AG, 3401 Burgdorf (CH)
(72) Inventor: Bosshard, Simon Martin, 3324 Hindelbank (CH); Zumstein, Dominik, 3014 Bern (CH); Hostettler, Patrick, 3415 Hasle (CH); Schrul, Christian, 3400 Burgdorf (CH); Bernhard, Mario, 3400 Burgdorf (CH)
(74) Representative: Meier Obertüfer, Jürg

(57) **Abstract**

An injection device for delivery of a fluid medicament comprising a housing (2) defining a longitudinal axis and enclosing an injection mechanism. An end cap (12) is fixated to the housing (2) and delimiting the injection mechanism in a proximal direction. A closure cap (17) is releasably connected to the end cap (12) by a releasable snap fit connection thereby closing the injection device. The releasable snap-fit connection is established before medicament delivery by relative axial movement along the longitudinal axis between the closure cap (17) and the housing (2) whereby a wall of the closure cap is elastically deformed, and the releasable snap-fit connection is configured to be released after medicament delivery by relative rotational movement around the longitudinal axis between the closure cap (17) and the housing (2) whereby the wall of the closure cap is elastically deformed.

## Description

### TECHNICAL FIELD

The current invention relates to an injection device comprising a releasable connection for a closure cap closing the injection device

### BACKGROUND OF THE INVENTION

Injection or infusion devices such as injections pens, auto injectors, autopens, patch injectors or patch pumps use a drive mechanism for expelling medicament from the device. The drive mechanism comprises mechanical components for advancing, for example, a piston rod forwarding a bung in a reservoir or to drive a pumping mechanism. The injection or infusion devices may be disposable or reusable devices. For disposable injection or infusion devices, the mechanical components are disposed after use and may be used for recycling specific components or the raw materials if the device is shredded. Alternatively, the device is combusted or ends at a waste depository.

There is a need to include additional features to those mechanical devices for example by adding additional modules to the mechanical device. Examples for those additional modules may be a connectivity module with a transmitter and/or receiver to allow communication with other devices, a sensing module to allow for sensing patient parameters such as blood glucose values, a location module indicating geographical locations, a timer or calendar module, a communication or training module including a loudspeaker, or a sensing module to detect impact. Those additional modules may be added as an add-on or supplementary device to the existing device. The add-on may be a separate add on that the end user attaches to the injection or infusion device or the add-on is integrated into the mechanical device by the device manufacturer, a so called integrated add-on.

The separate add on or the integrated add on may itself be disposable or reusable. For a reusable add on combined with a disposable injection or infusion device, the add-on must be removed from the disposable device for re-use of the add-on. For a disposable add on it may be beneficial to separate the add-on from the mechanical components for separate waste treatment as most add on devices include electronic components and batteries requiring a different treatment compared to the mechanical components.

There is a need to reduce waste and to facilitate re-use of components or materials or to recycle materials to reduce the amount of raw material or energy used throughout the lifecycle of the product. Thus there is a need to improve the injection device technologies including add-on features such that they are designed for recycling.

It is desired to encapsulate the modules comprising the add-on features in a housing part or closure cap that may be attached to the housing enclosing the mechanical components or to an end surface or end cap of the existing housing. The closure cap may protect the electronic components from contamination and mechanical damage. Additionally, the closure cap ensures that the user cannot see or touch the components enclosed in the closure cap.

After use, the add-on may be removed from the injection or infusion device such that mechanical and electronic components can be separated for recycling purposes. This will require removal of the closure cap and/or the electronic components enclosed in the closure cap.

In US7008399 an injection pen is disclosed comprising a printed circuit board (PCB), a battery and a LCD located behind a window in the dose setting knob. The mechanical and electronic components of the pen are fully integrated and there are no precautions taken to separate the components.

WO2018041798 discloses a reusable supplementary device that is releasable connected to the housing of an injection device. The mechanical connection uses snap fit connectors comprising hooks on flexible arms extending axially from the supplementary device engaging recesses in the housing. The mechanical connection is used for forming an optical coupling between the injection device and the supplementary device.

In EP 21187112.4 an autoinjector is disclosed with a module housing closing an electronic module that is releasable attached to a main housing comprising the injection mechanism. The module housing can be separated from the housing after injection.

It is an object of the present invention to improve the injection or infusion devices for recycling purposes by providing closure caps requiring less raw material that can be securely mounted onto the injection device before use and released after use. These objectives are solved by the independent claim and specific variants are disclosed in the dependent claims.

### DEFINITIONS

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

The distal end or distal direction is defined by the direction of the hollow needle configured to penetrate the skin of the patient. For an injection device such as an injection pen this may be the injection needle and the end of the pen holding the needle or being configured to hold the needle is the distal end. For an infusion device the distal end and the distal direction is towards the needle configured to penetrate the skin of the patient, which may be along the axis of the device or tilted or perpendicular to the axis of the device. The distal direction in an infusion device represents the direction in which the medicament flows towards the insertion needle. The proximal direction or end is opposite to the distal direction or end.

In the claims, the word "comprising" does not exclude other elements or steps, and the indefinite article "a" or "an" does not exclude a plurality. For example "a snap fit connector" does not exclude the fact that there may be two snap fit connectors that functionally or structurally fulfill the purpose of "a snap fit connector". The mere fact that certain elements or steps are recited in distinct claims shall not preclude the existence of further meaningful combinations of these elements or steps.

### GENERAL DESCRIPTION OF THE INVENTION

The current invention is for an injection device for delivery of a fluid medicament and the injection device comprises a housing defining a longitudinal axis and enclosing an injection mechanism. An end cap may be fixated to the housing thereby delimiting the injection mechanism in a proximal direction, and a closure cap may be releasably connected to the end cap thereby closing the injection device in the proximal direction. A releasable snap-fit connection may be located between the end cap and the closure cap and the releasable snap-fit connection is established before medicament delivery by relative axial movement along the longitudinal axis between the closure cap and the housing (or end cap) whereby a wall of the closure cap is elastically deformed. The releasable snap-fit connection may be configured to be released after medicament delivery by relative rotational movement around the longitudinal axis between the closure cap and the housing (or end cap) whereby the wall of the closure cap is elastically deformed.

The injection device may have an elongate housing and a closure cap for closing a proximal end of the injection device, the closure cap is mounted to the housing by relative axial movement along the longitudinal axis of the housing whereby a wall of the closure cap is elastically deformed. The wall of the closure cap is less rigid or more elastic compared to the housing such that the closure cap or a wall of the closure cap is deformed during the relative axial movement while the housing or the housing part engaging the closure cap preferably is not deformed or deformed to a lesser extent

The injection device may be a fix dose or a variable dose device such as an insulin pen or an autopen. The injection device may be an autoinjector using a prefilled syringe as a medicament holder. The injection device may be a disposable device or a re-usable device or a combination thereof, a so-called semi-disposable injection device. The injection device may also be an infusion device such as an infusion pump, a patch pump or a wearable bolus injector.

The housing may be composed of a single part or, preferably is an assembly of multiple parts that functionally and structurally behave as a housing. The housing may be an outer housing for protecting components from contamination. The housing may provide for mechanical support to other housing parts or to other mechanisms such as a dose setting mechanism, an injection mechanism, a needle insertion mechanism, a needle retraction mechanism, a needle cover mechanism and the like. The housing also encloses and preferably supports a reservoir and the injection mechanism is configured to deliver or expel doses from the reservoir that may have been set by a dose setting mechanism.

Part of the housing may be closed by caps, for example by a pen cap on one end and by the closure cap on the opposite end. The caps are preferably engaged with the housing by a releasable connection. The pen cap may be released by twisting or pulling the cap from the housing. The releasable connection may be released before or after expelling medicament from the injection device. The pen cap must be removed before expelling medicament as the injection needle must be capable for insertion into a patient as the pen cap covers the needle or the needle mounting unit. For an autoinjector comprising a prefilled syringe, the pen cap may also include a needle shield remover for simultaneous removal of the pen cap and the needle shield that covers and protects the needle before use. A pen cap may be part of a fully assembled device.

The injection mechanism in the housing is preferably closed by an end cap thereby delimiting or supporting the injection mechanism in the proximal direction. The end cap is preferably axially and rotationally secured to the housing. The housing and the end cap may structurally behave as a single part but are designed as separate parts facilitating assembly of the injection device. The end cap may be the proximal end of the device without an add-on module that may be optionally attached to the proximal end. The end cap may structurally support the housing and/or functionally contribute to the injection mechanism, for example by guiding parts of the injection mechanism or to provide visual, audible or tactile feedback.

The closure cap preferably closes the proximal end of the injection device opposite to the pen cap. The closure cap is attached to the housing or to a housing part, for example to the end cap of the housing. The closure cap preferably defines a space between the closure cap and the housing, more preferably between the closure cap and an end cap fixated to the housing. The end cap and the closure cap are preferably positioned both at the proximal end of the injection device or opposite to the end intended to be closed by the pen cap. The end cap may be the normal closing end for the injection mechanism and the additional closure cap may be added to or enclose an additional module for the injection device. The additional module may be an option that is added by the user or is already present in the device as manufactured. The modular approach increases the versatility of the injection device as one base unit (the housing closed by the end cap) may be combined with a plurality of modules. The additional module may be a mechanical module, an optical module, an electronic module comprising for example a transmitter/receiver, a computing device, a sensor such as a gravimetric sensor, a temperature sensor, a GPS device, a timer, a sim card, a device including a phone, a touch sensor, a capacitive sensor, a light detecting sensor, a battery package, a USB connector, a fluid control device, a switch or a switching device. The switching device may be used to activate components of the electronic module.

Preferably, the end cap closes the housing comprising mechanical components (the injection mechanism) whereas the closure cap encloses a module comprising non-mechanical (for example electronic) components as well as mechanical components (for example a switch). The electronic components are preferably located on a printed circuit board (PCB) which may be a rigid board or a flexible board.

Optionally, the outside surface of the closure cap has shock absorbing properties, for example to reduce the impact forces acting on the assembled injection device during mechanical impact such as during a drop-test. The shock absorbing properties may be due to additional ribs, protrusions or 3 dimensional structures extending outwards from the closure cap. For example a plurality of ribs may be spaced apart along the longitudinal axis and extend radially outward from the closure cap. Optionally, the closure cap is made from an elastic material or the closure cap is overmolded with an elastic material such as for example a thermoplastic elastomer.

As another option, the outside surface of the device housing or closure cap may also include a gripping surface, facilitating the handling or holding of the device by the user. The gripping surface may comprise ribs, grooves or an anatomic shape. The gripping surface may be made from another material that is added to or overmolded onto the closure cap. The material may be an elastomer, for example a thermoplastic elastomer. The gripping surface may be combined with the shock absorbing features mentioned above.

The outside surface of the closure cap may also include a clip for fixating the injection device to, for example, the clothing of a patient or a health care professional. The clip may be made from the same material as the cap (preferably a polymer) or from a different material such as a metal. The outside surface of the closure cap may also provide space for a label or QR code that is either directly printed onto the cap or the space is available for a separate label.

The surface of the closure cap may include visual aids. This may be a passive visual aid based on a fluorescent, a phosphorescent or luminescent material. An additive may be added to the material of a polymeric closure cap such that light is re-emitted during the night thereby guiding the user to find his injection device. Alternatively a separate layer of the material is added to the outside surface of the closure cap. Alternatively, an active visual aid is included to the closure cap or present within the closure cap such, for example, a LED light source. In an example, the closure cap or a part of the closure cap is made from a transparent or translucent material or a part is made from such a material and the LED is backlighting the closure cap. In another example, the wall of the cap is thin, for example below 0.5mm, and becomes translucent itself.

A releasable snap fit connection or snap closure or snap lock connection is defined as connection that, once established may be released. A snap-fit connection is formed between two parts that move relative to another in a first direction and the movement for forming the snap-fit connection may be different from a second direction for releasing the snap fit connection. Preferably the first and second directions are not opposite to another but at an angle to another, for example perpendicular to another. The snap fit connection may be configured to be released by a specific relative movement between the closure cap and the housing. For example a relative rotational movement or relative axial movement allows for the release of the connection whereas the other one of the axial or rotational movement is not configured for releasing the connection. The axial movement in one direction for forming the snap fit connection may not be released by a movement in the opposite axial direction whereas a rotational movement may release the connection. Optionally, the connection is formed by axial movement and released by a combination of axial and rotational movement. In the latter case either the closure cap is first axially moved to a release position whereby the closure cap is not released yet from the housing, followed by a rotational movement for releasing the closure cap from the injection device. The rotational movement may be in one rotational direction only or in both rotational directions. The closure cap may for example, first be rotated in a first rotation direction, subsequently axially moved to the aforementioned release position and finally the cap is released by rotation in the opposite rotation direction.

The releasable snap-fit connection is established before medicament delivery and preferably during assembly of the device, thus before an optional shelflife starts. Optionally, the releasable snap fit connection is formed just before medicament delivery, for example if the housing including the delivery mechanism and the closure cap are available as separate parts or as a kit of parts to the user or health care professional after an optional shelflife but before medicament delivery.

The relative axial movement along the longitudinal axis may be between the closure cap and the housing or between the closure cap and the end cap for the housing. The wall of the closure cap is elastically deformed. Preferably the wall of the closure cap excluding the snap-fit connector located on the inside or outside surface of the closure cap. Thus at least a part of the wall adjacent to the snap fit connector may elastically deform. The surface of the closure cap preferably does not contain flexible arms for forming the snap-fit connection as in the current invention, the wall or at least parts of the wall of the closure cap is elastically deformed. This leads to a robust snap-fit connection as thin flexible arms are avoided that may fracture. The relative axial movement between the closure cap and the housing may provide for audible or tactile feedback. Merging the closure cap to the housing may, next to forming the releasable snap fit connection, also form another connection, for example an electrical connection. Alternatively, the cap is attached by a relative axial movement combined with, or followed by a rotational closure movement. The closure movement is different from a rotational movement used for releasing the closure cap from the injection device. The closure movement may be part of a bayonet type of closure and the acoustic and/or tactile feedback may be specifically occur during the rotational closure movement after the initial axial movement.

The releasable snap-fit connection is configured to be released after medicament delivery by relative rotational movement around the longitudinal axis between the closure cap and the housing whereby the wall of the closure cap is elastically deformed. Different parts of the wall of the closure cap may be elastically deformed during axial merge and rotational release of the snap fit connection. Alternatively, the release of the snap fit connection by relative rotational movement results in a combination of elastic and plastic deformation of the closure cap.

The same snap fit connector or another part of the same snap fit connector or another snap fit connector on the closure cap and/or housing may be configured such that a relative rotational movement between the closure cap and the housing or the end cap releases the snap fit connection. The release of the snap fit connection intends to release the closure cap from the housing as two separate parts or two separate subassemblies. This may facilitate the separation between one subassembly comprising the mechanical parts and the other subassembly comprising electronic components.

Alternatively, the rotation of the closure cap triggers another dose setting step for the injection device, for example setting of a second or final dose after the first dose has been injected.

The relative rotational movement may trigger tactile and/or audible feedback to the user that is preferably different from the feedback for the axial movement. The release of the closure cap from the housing may also be used to disconnect an electrical contact, for example between the PCB and a battery.

The releasable snap-fit connection may comprise a snap fit connector on the end cap or housing comprising a first sloped surface. The releasable snap-fit connection may comprise a snap fit connector on the closure cap comprising a second sloped surface whereby the first and the second sloped surfaces are configured to be brought into a sliding engagement during relative movement along the longitudinal axis thereby elastically deforming the wall of the closure cap in a direction perpendicular to the longitudinal axis.

The snap fit connector on the closure cap may be located on an inside surface of the closure cap or on an outside surface of the closure cap. The complimentary snap fit connector on the end cap or on the housing may be located on an inside surface or an outside surface of the housing. The snap fit connector on the closure cap is preferably directly integrated into the wall of the closure cap and preferably there is no flexible arm between the closure cap and the snap fit connector. The snap fit connector on the closure cap is preferably located close to the distal end or distal rim of the cap whereas the snap fit connector on the housing or end cap is preferably located at the proximal end. The first and second sloped surfaces are preferably tilted with respect to the longitudinal axis. One or both of the surfaces may be flat, optionally one surface is flat whereas the other surface is curved.

A gearing arrangement may be formed upon mutual engagement of the first and second surfaces due to relative axial movement and the axial forces required for forming the snap fit connection are transferred in a force with a component directed perpendicular to the longitudinal axis. The perpendicular force component preferably results in elastic deformation of the closure cap or a wall of the closure cap. The deformation of the closure cap in the perpendicular direction may be due to a bending of the cap, preferably at the proximal end (close to the dome) of the closure cap.

The snap-fit connector on the end cap comprises a first stopping surface and the snap fit connector on the closure cap comprises a second stopping surface engaging the first stopping surface when the snap fit connection has been established thereby preventing the release of the releasable connection by relative axial movement between the closure cap and the end cap or housing.

The first and second stopping surfaces may be configured to prevent unintended release of the snap fit connection before medicament delivery. Preferably the first and second stopping surfaces are oriented essentially perpendicular to the longitudinal axis. There may be a single or a plurality of stopping surfaces on each snap fit connector. The snap fit connector or another snap fit connector on the end cap comprise the first stopping surface engaging the second stopping surface on the snap fit connector on the housing or on another snap fit connector on the housing or end cap. The presence of the stopping surface may prevent unintended release but also unintended re-use of a closure cap The snap-fit connector on the end cap or housing may comprise a third sloped surface oriented in the circumferential direction and the snap fit connector on the closure cap may comprise a complimentary fourth sloped surface, whereby the third and the fourth sloped surfaces are configured to be brought into a sliding engagement by relative rotational movement around the longitudinal axis between the closure cap and the housing thereby elastically deforming the wall of the closure cap in a direction perpendicular to the axis.

The third and fourth sloped surfaces may be on the snap fit connector on the end cap or housing and the closure cap respectively. Alternatively, the third and fourth sloped surfaces are on another snap fit connector on the end cap and closure cap respectively. The surface of the third and fourth sloped surface have a normal that is perpendicular to the longitudinal axis. The third and fourth sloped surface may be flat or curved or a combination thereof such that one of the third and fourth surfaces is essentially flat whereas the other one of the third and fourth surfaces is curved. Relative rotation between the closure cap and the housing or end cap may bring the third and fourth surface in a mutual gearing engagement with a force component oriented perpendicular to the longitudinal axis thereby elastically deforming the closure cap.

The rotational movement of the closure cap relative to the housing or end cap may be prevented by a separate rotation release mechanism or locking mechanism. Alternatively the rotation of the closure cap is prevented by the threshold that must be overcome for bringing the third and fourth sloped surfaces in an engagement together with the force needed for cap deformation.

The deformation of the closure cap in the perpendicular direction due to the rotational movement may be due to a bending of the cap, preferably at the proximal end (or dome) of the closure cap.

The closure cap may have a gripping surface that is preferably not located at the position of the snap fit connector on the closure cap. Preferably the gripping surface is at an offset angle to the snap fit connector on the closure cap. The gripping surface may have an additional purpose, namely to reduce the impact during a drop-test.

The first and the third sloped surface on the end cap are essentially oriented perpendicular to each other and the second and the fourth sloped surface on the closure cap are essentially oriented perpendicular to each other.

Preferably, the tilting axis of the first and the third sloped surfaces are oriented essentially perpendicular to another for the end cap and the tilting axis of the second and fourth sloped surfaces for the closure cap are oriented essentially perpendicular to another.

The injection device according to any of the previous claims wherein the end cap (or housing) and/or the snap fit connector on the end cap (or housing) has a stiffness that is greater than the stiffness of the wall of the closure cap.

The greater stiffness may be achieved by selecting a material with a higher stiffness for the housing or the end cap, or the greater stiffness may be achieved by locally increasing the thickness of the housing. The stiffness for the closure cap may be lowered by decreasing the thickness or selecting a material with a lower stiffness. Decreasing the thickness of the closure cap may be done locally. Selection of a material with a low stiffness may open a window for using recycled plastics or plastics that are polymerized from raw materials that are non-fossil based, for example biomass based. Alternatively, compostable plastics, for example starch based plastics may be used or biodegradable plastics may be used that are based on polylactic acid or copolymers containing polylactic acid (PLA, PLLA). These material may have a low stiffness which is an advantage for designing the closure cap and additionally, the cap is designed for recycling.

The thickness of the wall for the closure cap may be below 1 mm. Preferably, the thickness of the closure cap is below 0.9 mm, more preferably below 0.8 mm

At least a part of the closure cap is below 1 mm. For example the area surrounding the snap-fit connector on the closure cap is below 1 mm to facilitate the elastic deformation of the wall surrounding the connector. Alternatively, the proximal cap area or a dome area of the cap has a thickness below 1 mm. Preferably the snap fit connector on the closure cap has a thickness greater than 1 mm and is not elastically deformed, or at least to a lesser extend elastically deformed compared to the wall of the closure cap. The closure cap may have ribs located on the inside or outside which locally increase the thickness and therewith stiffness thereby guiding and controlling the elastic deformation of the closure cap.

The ribs may have a multiple purpose, either locally increasing the stiffness and/or providing a tactile surface for the user to grip and/or providing shock absorbing properties during impact.

The rotational movement of the closure cap relative to the housing or end cap may be prevented by a separate rotation release mechanism or locking mechanism comprising a separation bolt operatively coupled between the end cap or housing and the closure cap. The separate rotation release mechanism prevents unintended rotation of the closure cap and therewith unintended release of the closure cap before medicament delivery.

The coupling between the end cap (or housing) and the closure cap may prevent re-use of the closure cap once the closure cap has been released.

The separation bolt may be rotationally locked to the end cap or housing and rotationally locked to the closure cap by two form fit engagements and the separation bolt is configured to be rotationally unlocked from the end cap or housing by the injection mechanism.

The two form fit engagements may comprise a splined engagement between a key engaging a longitudinal slot. A splined engagement may exist between the closure cap and the separation bolt. A separate splined engagement may exist between the separation bolt and the end cap or the housing. The splined engagements are preferably oriented parallel to the longitudinal axis.

The injection mechanism may be configured to axially move the separation bolt to unlock the form fit engagement between the separation bolt and the end cap or housing.

The injection mechanism is delimited to the proximal end by the end cap and the injection mechanism may be capable to transmit a mechanical, an optical, a magnetic or an electrical signal via or through the end cap towards the closure cap and towards a module enclosed in the closure cap. The end cap itself may axially move or rotate for signal transmission or the end cap may, for example, comprise a passage, a pivoting arm, an electrically conductive area or a transparent window for signal transmission. Most preferably, the end cap comprises a passage allowing for a mechanical component to move through the end cap and thereby directly or indirectly axially move the separation bolt.

The injection mechanism for the injection device may comprise a holding element which is configured to be moved in the proximal direction by an injection spring upon medicament delivery and whereby the holding element is configured to move the separation bolt.

The injection device is preferably a spring driven device such as an autopen or an auto injector. Spring driven devices require means for holding the injection spring and/or a needle cover sleeve spring in a compressed state. The force of the injection spring may be released by the holding element thereby starting medicament delivery and the holding element may thereby be moved in the proximal direction towards the housing or to the end cap of the housing. Abutment of the holding element with the housing may result in tactile, audible or optical feedback indicating the start of the injection. Alternatively, another spring such as a needle cover sleeve spring may be tensioned before medicament delivery and the spring force may be released after medicament delivery and a signaling element is moved by the needle cover sleeve spring towards the housing or towards the end cap of the housing. The signaling element may abut the end cap to provide a tactile and/or audible signal thereby signaling that the injection has been completed. The movement of the holding element and/or the movement of the signaling element may move the separation bolt such that the form fit or splined engagement with the housing or end cap is released. The movement of the holding element and/or the movement of the signaling element may be used for actuation of an electronic module.

The movement of the signaling element or the holding element may be directly transmitted to the separation bolt, e.g. via a passage in the end cap or indirectly transmitted to the separation bolt, for example via a pivoting element that is part of the end cap. A pivoting element may, by virtue of the lever arm, increase the axial displacement of the separation bolt.

The injection device may be subjected to impact forces and those impact forces may lead to unintentional movement of the holding element and/or the signaling element. An additional securing mechanism may be included in the end cap preventing unintentional movement of the holding element or signaling element. The securing mechanism may have an elastic arm biasing the holding element towards the distal end of the injection device providing a threshold for movement into the proximal direction.

The injection spring for the injection device may be kept in a compressed state by the holding element before medicament delivery and the injection spring may be configured to advance a plunger rod for medicament delivery when the holding element is released.

Preferably, the injection spring is operatively positioned between the holding element and the plunger rod. The ends of the injection spring may act on the holding element and on an internal surface of the plunger rod. Before injection, the injection spring is kept in a compressed state by a form fit engagement between the holding element and the plunger rod which is released when the injection is started. The form fit engagement may be released by movement of a needle cover sleeve. The plunger rod moves in the distal direction for medicament delivery and the holding element moves in the proximal direction providing the start of injection click and/or releasing the separation bolt. In case the movement of the signaling element is used for moving the separation bolt then the bolt is moved after or during the end-of-delivery click.

The closure cap for the injection device may comprise a protrusion or guide pin engaging a guide slot on the end-cap or housing thereby limiting the maximum rotation of the closure cap with respect to the end cap or housing. The guide pin may extend axially along the longitudinal axis or may be tilted with respect to the axis.

Rotation of the guide pin in the guide slot may be used to move the closure cap in the proximal direction with respect to the housing after the snap fit connection has been released. The maximum rotation may be limited in one or both rotation directions. The guide slot may be a linear guide slot or a tilted guide slot or a combination thereof to form a link-motion engagement. A stopping surface on the guide pin abuts the end of the guide slot preventing further rotation of the closure cap.

The closure cap defines a space between the closure cap and the end cap and an electronic module including a printed circuit board and a battery may be located within the space.

Preferably, the electronic module comprises at least one switch that may be activated by the holding element or signaling element. The electronic module may comprise a battery and/or a transmitter/receiver and/or an antenna. The electronic module may be in a sleeping mode before activation and the switch may wake up the electronic module. The electronic module may have a second switch that is activated during rotation and removal of the closure cap indicating that the cap has been successfully removed.Alternatively, the switch that is activated by the holding element or the signaling element is also used, activated, deactivated during rotation of the closure cap.

The electronic module may be fixated to the closure cap by relative rotation between the closure cap and the end cap. Preferably, the electronic module or the printed circuit board is not fixated or only partially fixated to the closure cap before medicament delivery.

The electronic module, preferably the PCB including all the electronic components may be fixated to the closure cap due to the rotational movement such that the closure cap can be removed together with the electronic module for recycling purposes. Preferably, the snap fit connection between the closure cap and the end cap is released before fixating the closure cap to the PCB. Fixating elements on the closure cap and on the PCB may be brought into engagement upon rotation of the closure cap thereby axially and rotationally fixating the PCB to the closure cap.

In a further aspect which is not part of the current invention, the injection device comprises a housing or housing part defining a longitudinal axis enclosing an injection mechanism. The housing part may be the end cap described above. The injection device may be closed by a closure cap being releasable connectable or connected to the housing or housing part by a release mechanism. The injection device may have a locking mechanism for the release mechanism, the locking mechanism comprises a locking element, preferably the separation bolt described above. The locking element may be configured to be moved along the longitudinal axis between a first position (preferably before injection) keeping the release mechanism in the locked position and a second position (preferably after injection) thereby unlocking the release mechanism. The locking mechanism is characterized by a securing element which is configured to bias the locking element towards the first position.

The release mechanism is configured to release and separate the closure cap from the injection device and can be moved from the locked position to the unlocked position, preferably by rotation of the closure cap. The release mechanism is located or operatively coupled between the closure cap and the housing or housing part such as the end cap for the injection mechanism. The end cap preferably delimits the injection mechanism.

The locking mechanism is configured to keep the release mechanism in the locked position until the injection mechanism has been used and medicament has been expelled. During or after medicament delivery, the locking mechanism unlocks the release mechanism such that the release mechanism can be moved to the unlocked position. In the unlocked position the closure cap may be removed from the injection device.

The securing element secures the locking element (or separation bolt) in the first position and a threshold in terms of force needs to be overcome before the locking element can move towards the second position. The movement from the first to the second position is intended to be dictated by medicament delivery. Before medicament delivery, unintended movement of the locking element, for example due to impact forces needs to be avoided as this may lead to unintended release of the release mechanism and therewith unintended removal of the closure cap.

The securing element may be part of, or connected to the housing or housing part of the injection device and comprises a flexible arm biased towards the longitudinal axis which is configured to bias the locking element towards the first position.

Alternative to the flexible arm, the securing element may comprise a spring, a compression spring or leg spring, or a magnetic or an electromagnetic coupling. Alternatively to the biasing direction towards the longitudinal axis (or center of the device), the biasing direction may be along the longitudinal axis, preferably in the distal direction.

The securing element may act directly on the locking element or indirectly via other parts and may comprise a lever arm.

The flexible arm of the securing element may comprise a first surface configured to engage the locking element and wherein movement of the locking element from the first position towards the second position elastically deforms the flexible arm away from the longitudinal axis against the bias.

The bias of the flexible arm may be converted in an axial bias or force using the first surface on the flexible arm contacting the locking element. The engagement between the first surface on the flexible arm of the securing element and the locking element may be that the locking element has a counter surface or a point of contact. The surfaces on the flexible arm and optionally on the locking element may be flat or curved. The elastic deformation provides the threshold that must to be overcome before the locking element can move to or towards the second position for unlocking the release mechanism thereby increasing the reliability of the device, for example the impact resistance.

Preferably the locking element or separation bolt comprises a third surface that is preferably complementary to the first surface on the flexible arm of the securing element. The first and third surfaces preferably form a gearing once engaged due to the movement of the locking element and/or the bias of the flexible arm. The gearing preferably transfers the radial bias of the flexible arm into a longitudinal bias biasing the locking element towards the first position, preferably towards the distal end of the injection or infusion device.

When the locking element moves towards the second position, the bias towards the first position may gradually increase due to flexing of the arm or compression of the spring in combination with the surfaces that slide over each other.

There may be a single arm or a plurality of flexible arms and all the arms may simultaneously engage the locking element with the first surfaces. Alternatively, the surfaces of the plurality of flexible arms may consecutively engage the locking element.

The flexible arm of the securing element may comprise a second surface configured to engage the locking element when the locking element moves towards the second position, preferably after first being moved out of the first position.

The axial hub of the locking element may be limited, or there may be a large variation in the length of the axial hub due to tolerances within the injection mechanism. Therefore it may be beneficial to enhance the movement towards the second position once the threshold biasing the locking element towards the first position has been reached. The movement towards the second position may be enhanced by the securing element to enlarge the axial hub for the locking element thereby enhancing the reliability of the injection device comprising the securing element.

Preferably, the second surface of the securing element is configured to engage a fourth surface on the locking element and wherein a gearing is formed between the second and fourth surfaces biased by the flexible arm thereby supporting the movement of the locking element towards the second position, preferably after first being biased towards the first position. The second and fourth surfaces are preferably complementary surfaces and may be flat surfaces or only one of the two surfaces is a flat surface abutting a curved surface or only a point of contact on the counterpart.

The gearing formed between the first and third surface and the gearing formed between the second and fourth surface are both biased by the flexible arm of the securing element. The biased gearing between the first and third surface is preferably directed in a direction opposite to the direction of the gearing between the second and fourth surfaces. The gearings preferably have, once biased, a component directed along the longitudinal axis of the injection device. The force component along the longitudinal axis for the gearing between the first and third surfaces is preferably directed towards the distal direction whereas the gearing between the second and fourth surfaces has a proximal force component. The gearing between the second and fourth surface is preferably formed after release of the gearing between the first and third surfaces.

Preferably the securing element comprises a crest between the first and second surfaces, the first surface is configured to bias the locking element towards the first position whereas the second surface is configured to bias the locking element towards the second position.

Once a threshold bias towards the first position of the locking element has been overcome then the bias of the flexible arm with the gearing between the second and fourth surfaces biases the locking element towards the second position. Overcoming the threshold may increase the bias for moving the locking element towards the second position as the flexing of the arm of the securing element gradually increases when moving out of the first position towards the second position.

The housing or housing part of the injection device encloses an injection mechanism and the injection mechanism is configured to move the locking element from the first to the second position against the initial bias provided by the securing element.

The injection mechanism preferably comprises an injection spring which is configured for advancing a piston rod and wherein the injection spring is kept in the compressed state before medicament delivery by the holding element. Alternatively and optionally, the needle cover sleeve spring biases another element in the injection mechanism, for example a signaling element.

The holding element may be released for medicament delivery thereby releasing the injection spring for advancing the piston rod and the holding element is thereby moved by the injection spring in a proximal direction and may abut and move the locking element. Alternatively, the needle cover spring sleeve is tensioned before or during an injection and biases the signaling element towards the end cap of the housing and/or the locking element for the release mechanism. Movement of the locking element, for example for producing an end of injection click, may be used for moving the locking element.Preferably, the closure cap may be released from the injection device by rotation of the closure cap relative to the housing after unlocking the release mechanism by the locking mechanism and thus preferably after medicament delivery.

The closure cap is preferably rotationally locked to the locking element (or separation bolt).

The locking element or separation bolt is preferably rotationally locked to the housing when the locking element is in the first position thereby preventing rotation of the locking element relative to the housing and preventing rotation of the closure cap relative to the housing.

Rotation of the closure cap relative to the housing preferably fixates a printed circuit board to the closure cap. The closure cap may enclose an electronic module comprising a printed circuit board. Rotation of the closure cap relative to the housing may fixate the PCB to the closure cap and rotation of the closure cap may release the closure cap from the housing together with the PCB.

Rotation of the closure cap relative to the housing may elastically deform the closure cap thereby releasing a snap fit connection between the housing and the closure cap such that the closure cap may be removed from the housing.

### LEGENDS TO THE FIGURES

While the invention has been described in detail in the drawings below and foregoing general description, such description is to be considered illustrative or exemplary and not restrictive. Variations to the disclosed embodiments can be understood and effected by those skilled in the art and practicing the claimed invention, from a study of the drawings, the disclosure, and the appended claims.
- Figure 1:: Exploded view for an autoinjector,
- Figure 2a:: Longitudinal section for the autoinjector,
- Figure 2b:: Longitudinal section for the autoinjector, in a view perpendicular to Figure 2a,
- Figure 3a:: Longitudinal section of the proximal part of the autoinjector showing an electronic module in a closure cap and the locking mechanism for the release mechanism of the closure cap. Separation bolt in first position,
- Figure 3b:: Longitudinal section of the proximal part in a view perpendicular to Figure 3a,
- Figure 4a:: Longitudinal section of the proximal part of the autoinjector showing an electronic module in a closure cap and the locking mechanism for the release mechanism of the closure cap. Separation bolt in second position,
- Figure 4b:: Longitudinal section of the proximal part in a view perpendicular to Figure 4a,
- Figure 4c:: Detail of the engagement between the securing element and the locking element (or separation bolt),
- Figure 5a:: Axial mounting of the closure cap, snap fit connectors on the closure cap and on the end cap not engaged,
- Figure 5b:: Axial mounting of the closure cap, snap fit connectors on the closure cap and on the end cap partially engaged,
- Figure 5c:: Axial mounting of the closure cap, closure cap fully engaged and the locking mechanism in the locked position,
- Figure 6a:: End cap,
- Figure 6b:: End cap,
- Figure 7a:: Mounting of the closure cap to the end cap shown in Figure 6a,
- Figure 7b:: Mounting of the closure cap to the end cap shown in Figure 6b,
- Figure 8a:: Assembly of end cap and closure cap before relative rotation,
- Figure 8b:: Assembly of end cap and closure cap after relative rotation,
- Figure 9a:: Cross section in a plane perpendicular to the longitudinal axis showing the snap fit connectors of the closure cap and the end cap together with a top view on the separation bolt. Separation bolt in the locked position,
- Figure 9b:: Cross section in a plane perpendicular to the longitudinal axis showing the snap fit connectors of the closure cap and the end cap together with a top view on the separation bolt. Separation bolt in locked position showing the engagement between the separation bolt and the closure cap,
- Figure 9c:: Cross section in a plane perpendicular to the longitudinal axis showing the snap fit connectors of the closure cap and the end cap together with a top view on the separation bolt. Separation bolt released from housing and closure cap partially twisted,
- Figure 9d:: Cross section in a plane perpendicular to the longitudinal axis showing the snap fit connectors of the closure cap and the end cap together with a top view on the separation bolt. Separation bolt released from housing and closure cap fully twisted for release of the closure cap,
- Figure 10a:: Assembly of the closure cap and the PCB before rotation of the closure cap,
- Figure 10b:: Assembly of the closure cap and the PCB before rotation of the closure cap,
- Figure 11a:: Assembly of the closure cap and the PCB after rotation of the closure cap,
- Figure 11b:: Assembly of the closure cap and the PCB after rotation of the closure cap,
- Figure 12a:: Alternative embodiment for connector on the PCB
- Figure 12b:: Alternative embodiment for connectors between the closure cap and the PCB,
- Figure 13a:: Alternative embodiment for connector on the PCB
- Figure 13b:: Alternative embodiment for connectors between the closure cap and the PCB.

### DETAILED DESCRIPTION OF THE FIGURES

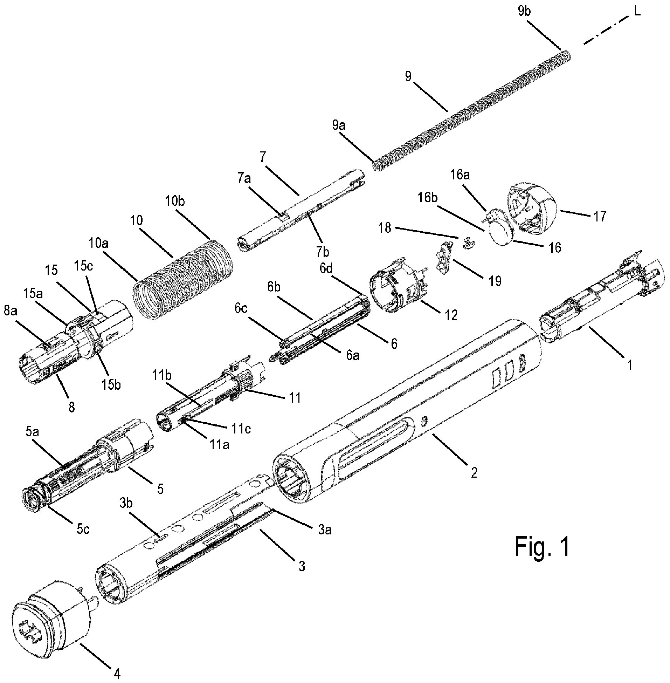

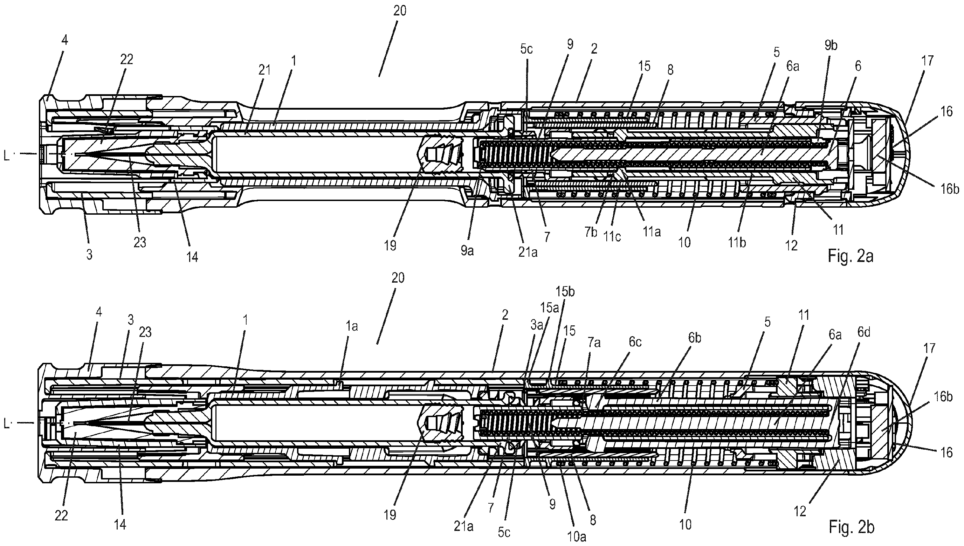

Figure 1 presents an exploded view for an autoinjector 20 and Figures 2a and 2b longitudinal sections for the autoinjector 20. An elongated sleeve shaped housing 2 can be gripped by the user during an injection and the housing defines a longitudinal axis L. An injection mechanism that is configured to be driven by a coiled compression spring is enclosed in the housing 2. A prefilled syringe 21 (Figure 2) is received in a syringe holder 1 and the syringe holder 1 is axially and rotationally secured to the housing 2 with a snap-fit connection. The autoinjector 20 as delivered to the patient is closed at the distal end by a pen cap 4 that must be removed before using the autoinjector. A needle shield 22 of the prefilled syringe 21 is coupled to the cap 4 by a needle shield remover 14 such that removal of the pen cap 4 also removes the needle shield 22 thereby exposing hollow needle 23. A needle cover sleeve 3 is axially guided by, and can be moved relative to the housing 2 along the longitudinal axis L a distance corresponding to an actuation hub. The needle cover sleeve 3 can slide in the proximal direction for actuating the injection mechanism to expel medicament from the autoinjector 20. A mechanic holder 5 is snap-fitted into and rotationally and axially secured to the housing 2. The mechanic holder 5 comprises an elastic element 5c at the distal end which abuts the proximal end 21a of the prefilled syringe 21 and biases the prefilled syringe distally into the syringe holder 1.

The injection mechanism comprises a compression spring acting as an injection spring 9. The injection spring 9 in its initial state is almost completely enclosed by a piston rod 7 and the distal end 9a of the injection spring 9 biases the piston rod 7 into the distal direction. The proximal end 9b of the injection spring abuts a holding element 6 and the holding element 6 comprises two arms 6b extending in the distal direction and one central pin 6a for guiding the injection spring 9. Each arm 6b comprises a protrusion 6c directed towards the axis L and both protrusions engage recesses 7a on the outer surface of the piston rod 7. In the initial state of the autoinjector, a switching module 8, 15 comprising switching sleeve 15 and a locking sleeve 8 prevent a deflection of the arms 6b as the arms 6b are confined within the locking sleeve 8 and therewith prevent a relative movement between the piston rod 7 and the holding element 6 thus keeping the injection spring 9 in a compressed state. The piston rod 7 is prevented from movement into the distal direction. At least the distal end 15a of the switching sleeve 15 is engaged or abuts with a proximal end 3a of the needle cover sleeve 3. The switching sleeve 15 is biased by a needle cover sleeve spring 10 into the distal direction. The distal end 10a of the needle cover sleeve spring 10 abuts a rim 15b on the switching sleeve 15 and the proximal end 10b of the needle cover sleeve spring 10 abuts a signaling element 11 and the signaling element abuts an end cap 12 that is axially and rotationally secured to the housing 2. In the initial state of the autoinjector 20, the force of the needle cover sleeve spring 10 in the proximal direction is guided to the end cap 12 of the housing 2 via the signaling element 11. In the distal direction, the needle cover sleeve spring 10 biases the needle cover sleeve 3 into the distal direction via the switching sleeve 15. For starting the injection, the needle cover sleeve 3 is pushed against the skin of the patient and moved in the proximal direction with respect to the housing 2 thereby moving the switching module 15, 8 in the proximal direction and tensioning the needle cover sleeve spring 10. In the most proximal position of the switching module 15, 8, the locking sleeve 8 may be axially engaged with the mechanic holder 5 (or alternatively to the signaling element 11 as will be discussed below) whereas the switching sleeve 15, axially guided and splined onto the locking sleeve 8, is kept by the needle cover sleeve 3 in the proximal position against the bias of the spring force provided by the needle cover sleeve spring 10.

The proximal movement of the switching module 8, 15 releases the engagement between the protrusions 6c on the holding element 6 and the recesses 7a in the piston rod 7. The spring force is released and the piston rod 7 moves into the distal direction whereas the holding element 6 can move an initial hub in the proximal direction until the holding element 6 abuts the end cap 12 of the housing 2. Optionally, an acoustic or tactile signal is generated upon abutment indicating the start of the injection. The piston rod 7 abuts a bung 19 in the prefilled syringe which is moved distally towards the outlet thereby expelling medicament through the needle 23.

The needle cover sleeve spring 10 is a metal coil spring acting as a compression spring and the proximal end 10b abuts the signaling element 11 which abuts the end cap 12 of the housing 2 before injection. The signaling element 11 comprises two longitudinally extending arms 11b each having a protrusion 11a oriented towards the longitudinal axis L. The protrusions 11a engage another recesses 7b in the piston rod 7 and the sleeves of the switching module 8, 15 ensure that the signaling element 11 is axially coupled to the piston rod 7 before and during the initial stage of the injection. The arms 11b with protrusions 11a are also confined within the locking sleeve 8 thereby preventing release of the protrusions from the recesses. During the initial stage of the injection, the piston rod 7 is advanced distally and thereby also drives the signaling element 11 in the distal direction away from the end cap 12 through the locking sleeve 8. The signaling element 11 is moved in the distal direction by a distance or tensioning hub until the protrusions 11a are released from the recesses 7b in the piston rod 7 as the arms 11b are not confined by the locking sleeve 8 anymore and can flex radially outward. The arms 11b have another protrusion 11c that is directed in the radial outward direction and those outward protrusions 11c engage the switching module 8, 15 keeping the tensioning hub of the signaling element 11. The arms 11c preferably engage the locking sleeve 8 thereby keeping the locking sleeve 8 in the proximal position (alternatively the locking sleeve engages the mechanic holder 5 as discussed above). The arms 11b can slide along the outer surface of the piston rod 7 and at the end of the injection, the piston rod has advanced such that the arms 1 1b of the signaling element 11 can flex radially inward again and the signaling element 11 is released from the switching module 8, 15. The signaling element 11 is biased proximally by the needle cover sleeve spring 10 and is accelerated and moved back towards the end cap 12 thereby producing an audible and/or tactile end-of-injection click.

After the medicament has been delivered, the autoinjector 20 is removed from the skin and the switching sleeve 15 together with the needle cover sleeve 3 are forwarded by the needle cover sleeve spring 10 that is at least partially decompressed. The switching sleeve 15 moves in the distal direction and is sliding along the locking sleeve 8 which has been locked before injection to the mechanic holder 5. The switching sleeve 15 may be locked in the most distal position by a flexible arm 8a of the locking sleeve 8 engaging a cut out 15c of the switching sleeve 15 thereby preventing proximal movement of the switching sleeve 15 after injection. The needle cover sleeve 3 may be locked in the most distal position by arms 1a of the syringe holder 1 that radially extend into recesses 3b of the needle cover sleeve 3 thereby preventing proximal movement of the needle cover sleeve 3 after injection.

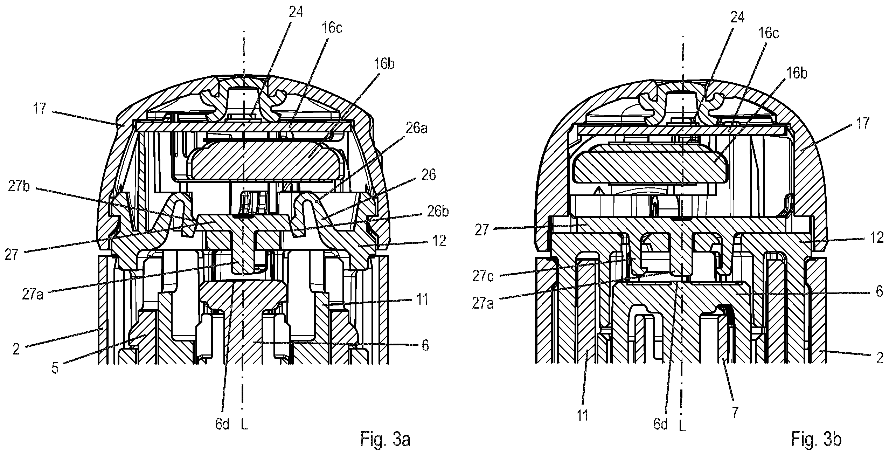

The end cap 12 is axially and rotationally fixed to the housing 2. The end cap 12 may be snap fitted, adhesively connected or welded to the housing 2. The end cap 12 can be the closure cap for the autoinjector delimiting the mechanical parts of the device in the proximal direction if there are no further modules added to the autoinjector. The end cap may provide protrusions, ribs or 3 D structures acting as shock absorbers. Preferably, the end cap 12 provides a platform or adapter for adding an additional module to the autoinjector. Preferably this is an electronic module 16 and the module is closed by a closure cap 17 engaging the end cap 12 and/or housing 2 of the autoinjector. The closure cap 17 may provide protrusions, ribs or 3 D structures on the outer surface acting as shock absorbers. Preferably, the module is a releasable module that can be released and separated from the autoinjector after injection. The electronic module 16 may comprise a sensor or switch 16a and a battery 16b and a printed circuit board 16c. The printed circuit board 16c may comprise a microprocessor, a transmitter/receiver unit, an antenna and/or a light source such as a LED 24 (Figure 3a). The light source may be used to signal the status of the device to the user, for example "ready to use" or "injection completed". The sensor 16a may be part of the PCB 16c as well and is configured to detect if the signaling element 11 is in the most proximal position. The sensor 16a may be a switch located on the PCB 16c detecting whether the signaling element 11 is in its most proximal position before injection and/or the most proximal position after injection and/or the absence of contact to the sensor during an injection. The signaling element 11 may have a protrusion extending into the proximal direction and optionally guided through passage in the end cap 12 for contacting the sensor 16a. Alternatively, the switching element indirectly contacts the sensor 16a, for example via a pivoting element or rotating element 18 that is part of or coupled to the end cap 12. The microprocessor detects the signals from the sensor 16a and is capable to transmit the data (for example time, duration, failure) of an injection to an external device using a communication module comprising, for example a transmitter and/or receiver.

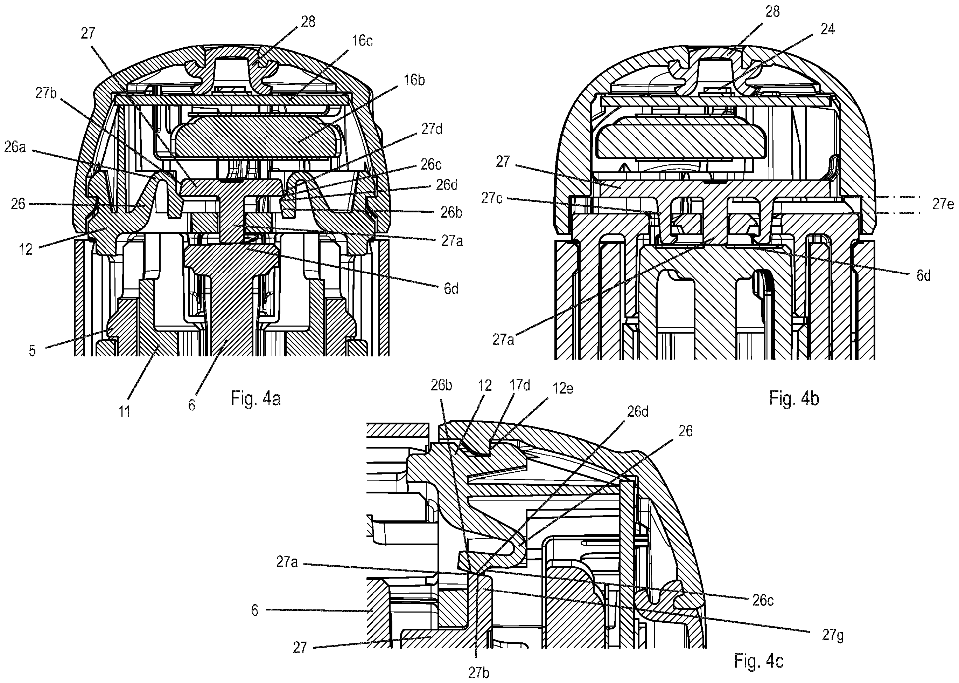

The closure cap 17 for the electronic module is preferably releasable connected to the housing 2 and/or to the end cap 12. A locking mechanism keeps the releasable connection in a locked position and the locking mechanism may be unlocked by the injection mechanism. Details are shown in Figures 3a, 3b where the locking mechanism is in the locked position, and in Figures 4a, 4b and 4c for the unlocked position.

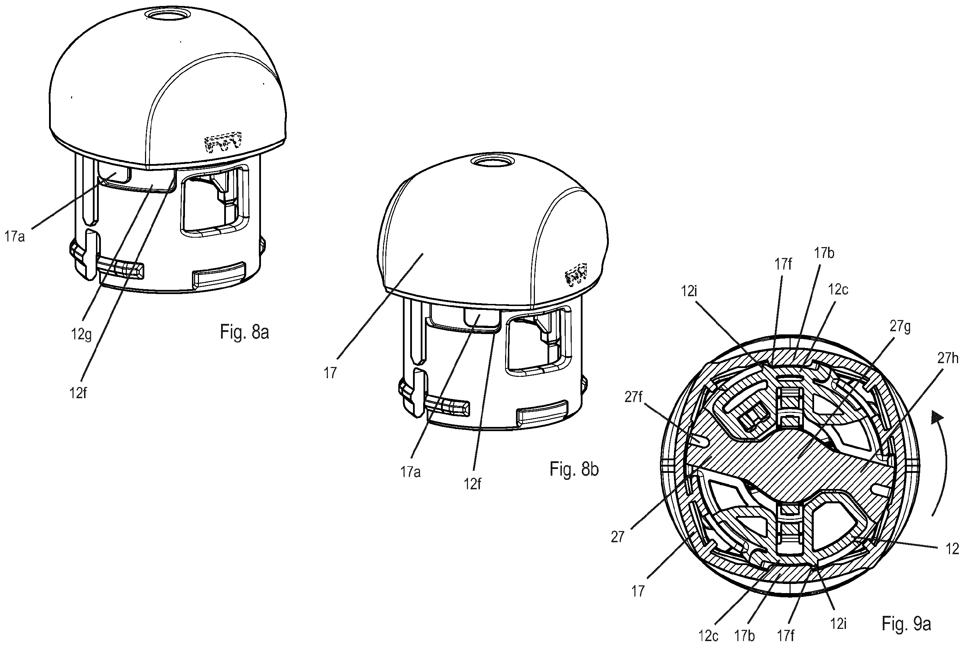

Figure 3a presents the closure cap 17 attached to the end cap 12 thereby being axially and rotationally coupled to the housing 2. The closure cap 17 encloses the printed circuit board (PCB) 16c with the battery 16b. The end cap 12 comprises a securing element 26 in the example shown in Figure 3a. Alternatively, the housing 2 comprises the securing element 26. The securing element 26 may be attached to the end cap 12 as a separate element or, preferably, the securing element 26 is integrated into the end cap 12, for example the securing element is injection molded together with the end cap 12 as a single unit. The securing element may comprise a plurality of flexible arms 26a having a U-shaped section and the end of each flexible arm 26a points towards the longitudinal axis L of the autoinjector. The flexible arms 26a may provide a bias towards the longitudinal axis when the arms are elastically deformed. More particularly once the legs of the U-shaped section are compressed towards each other. At the end of the flexible arm 26a there are two sloped surfaces 26b, 26c with a crest 26d between the two sloped surfaces (details are shown in Figure 4c). The surfaces 26b, 26c are inclined with respect to the longitudinal axis L. A locking element 27, also called separation bolt, is positioned between the electronic module and the end cap 12. The separation bolt 27 comprises wings 27h (Figure 9a) that extends radially along the end cap 12 and a protrusion 27a that extends axially along the longitudinal axis L through the end cap 12 in the distal direction (Figure 3b). Arms 27c surround the central protrusion 27a and protrude through openings in the end cap 12. Hooks at the end of the arms 27c ensure that the separation bolt 27 is axially secured to the end cap 12 while allowing for relative limited axial movement and rotation between the end cap 12 and the separation bolt 27. Figures 3a and 3b present the autoinjector before medicament delivery and the signaling element 11 abuts the end cap 12. The proximal end 6d of the holding element 6 is separated from the protrusion 27a extending from the separation bolt 27. The separation bolt 27 comprises a central disk shaped portion 27g comprising wings 27h that extend in the radial direction and the protrusion 27a extends axially from the disk shaped portion (Figures 3a, 9a). The disk 27g comprises a third sloped surface 27b at an edge of the separation bolt 27 and is being configured to engage the first sloped surface 26b on the securing element 26 before injection starts (Figures 3a, 4c). The engagement 26b, 27b biases the separation bolt 27 in the distal direction towards a first position. During start of the injection, the holding element 6 is moved in the proximal direction and the proximal end 6d of the holding element 6 abuts the separation bolt 27 such that the first surface 26b and the third surface 27b engage each other and form a gearing such that the sliding of the third surface 27b of the separation bolt 27 against the first surface 26b of the securing element 26 provide for a force that is directed radially outward thus flexing and tensioning the arm 26a. The bias of the tensioned arm with the gearing 26b, 27b tends to move the separation bolt 27 in the distal direction. As the holding element 6 continues to push on the separation bolt and surpasses a threshold, the third surface 27b passes the crest 26d on the flexible arm 26. The flexible arm 26a relaxes back to its original position as the second surface 26c engages the fourth surface 27d (or in this example point of contact) on the edge of the disk shaped portion 27g of the separation bolt 27. For details see Figure 4c. The engagement between the fourth surface 27d and the second surface 26c forms a gearing and the relaxation of the elastic energy stored in the flexible arms 26a pushes the separation bolt 27 towards the second position or the unlocked position. The separation bolt 27 can be lifted a distance indicated by gap 27e (Figure 4b). Lifting the separation bolt 27 releases a form fit engagement between the separation bolt 27 and the housing 2 or end cap 12 as will be shown below and therewith allows for rotation of the separation bolt and closure cap with respect to the housing. The closure cap 17 may be released from the housing as the locking mechanism with the locking element (or separation bolt) has been moved to the unlocked position.

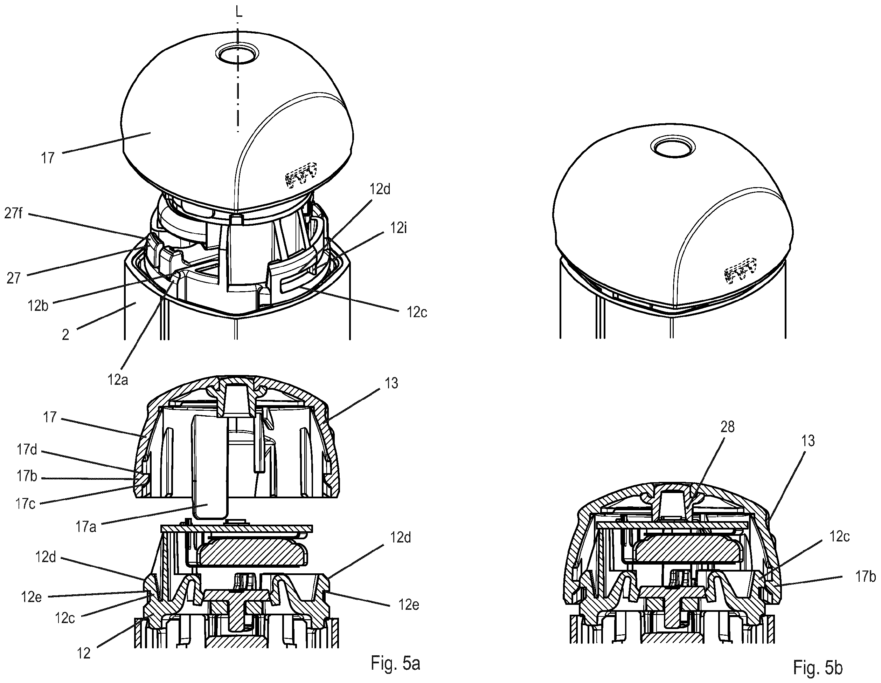

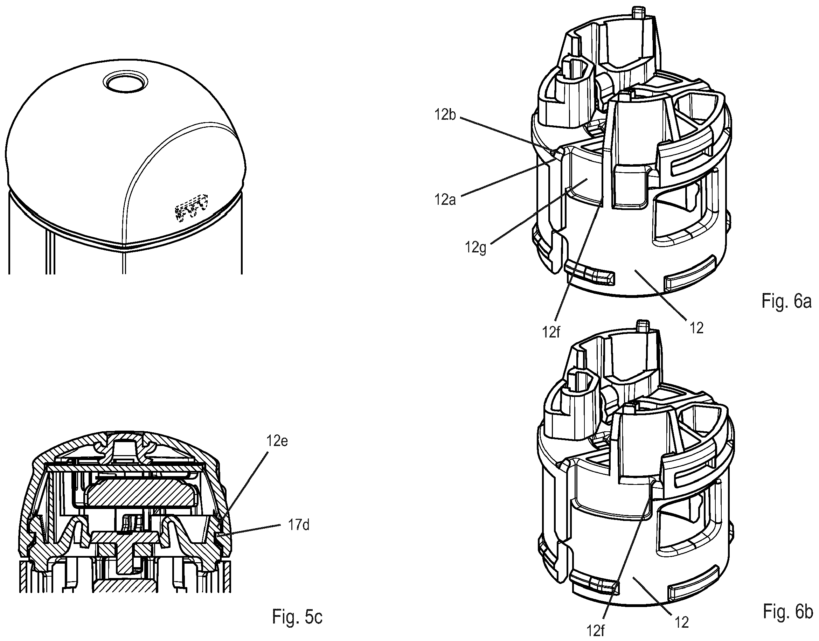

The mounting of a preferably thin walled closure cap will be described in the following (Figures 5a, 5b and 5c). The wall 13 of the closure cap that is adjacent to snap fit connectors described below has a thickness below 1 mm preferably below 0.8 mm. The closure cap 17 is moved along the longitudinal axis L towards the end cap 12 or the housing 2 (Figure 5a). The end cap 12 comprises at least one snap fit connector 12c comprising a first sloped surface 12d which is tilted with respect to the longitudinal axis L and a first stopping surface 12e oriented essentially perpendicular to the longitudinal axis L. The closure cap 17 comprises at least one snap fit connector 17b having a second sloped surface 17c complimentary to the first sloped surface 12d on the snap fit connector 12c on the end cap 12. The snap fit connector 17b comprises a second stopping surface 17d configured to engage the first stopping surface 12e on the end cap 12. The first and second sloped surfaces 12d, 17c slide over each other as the closure cap 17 approaches the end cap 12 and the engagement between the sloped surfaces 12d, 17c results in a force directed radially outward which elastically deforms the preferably thin walled closure cap 17 (Figure 5b). The stopping surfaces 12e, 17d engage each other as the closure cap has reached its final axial position and the closure cap can elastically relax back to its initial position (Figure 5c). The engagement of the stopping surfaces 12e, 17d prevent axial rearward movement of the closure cap 17 and thus unintended release of the closure cap from the end cap before injection.

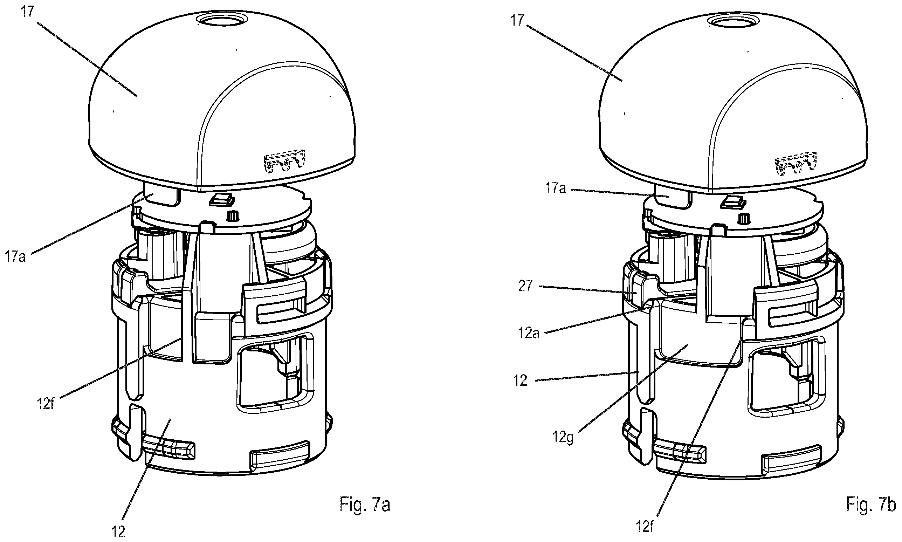

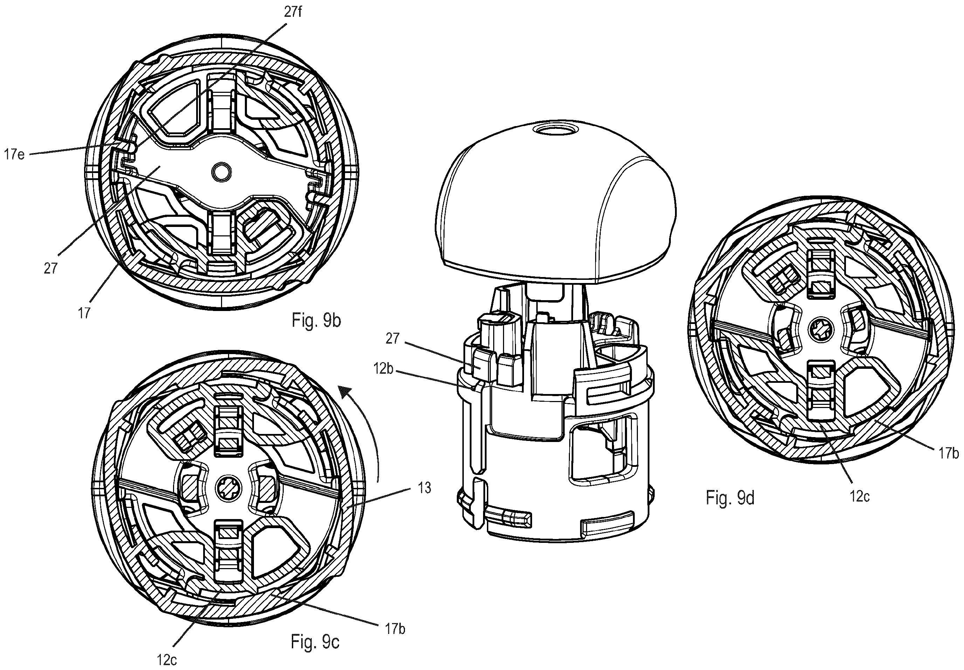

Rotation of the closure cap 17 with respect to the housing or end cap 12 is prevented before releasing the locking mechanism by the injection mechanism. The closure cap 12 is rotationally locked to the separation bolt 27 as the wings 27h comprise a groove 27f that extends along the axis L and the grooves 27f engage a longitudinal key 17e on the closure cap (Figures 5a, 9a, 9b). A radial stop 12a on the end cap 12 abuts an edge of the wings 27h (Figure 7b) thereby preventing rotation of the separation bolt 27 and thus also rotation of the closure cap 17 via the key/groove engagement 27f, 17e (Figure 9b). Once the separation bolt 27 has been lifted by the injection mechanism a distance 27e exceeding step 12b, then the separation bolt 27 and therewith also the closure cap 17 may rotate (Figure 9d).

The maximum rotation of the closure cap may be limited by a radial stop 12f engaging a protrusion 17a axially extending from the closure cap 17. The end cap 12 may form a guide slot 12g for the protrusion. The position of the radial stop 12f defines the maximum angle of rotation. In Figure 6a, the radial stop 12f is positioned such that the protrusion 17a snuggly fits in thereby allowing no or only limited rotation of the closure cap (Figure 7a). In Figure 6b, the protrusion may rotate a certain angle until the protrusion 17a abuts radial stop 12f (Figure 7b). Figures 6a and 6b show two different embodiments for the end cap 12 with a different position of the radial stop 12f. The end cap is injection molded and the two versions can be made from one modular tooling by simply removing or adding one part to the mold.

The closure cap 17 may be released from the housing 2 and/or end cap 12 by relative rotation between the closure cap 17 and the housing (preferably after the injection mechanism has released the locking mechanism for the separation bolt). The assembly of a closure cap 17 that cannot rotate with respect to the end cap 12 is presented in Figure 7a. The protrusion 17a fits into and abuts the radial stop 12f preventing relative rotation between the two caps. Figures 7b, 8a and 8b represent an assembly of the closure cap 17 that may rotate with respect to the end cap once the locking mechanism has been released. The protrusion 17a may move in the guide slot 12g until the protrusion abuts the radial stop 12f (Figure 8b). The rotation of the closure cap 17 is transferred into a rotation of the separation bolt or locking element 27 via the key/groove interaction 27f, 17e (Figure 9b).

Rotation of the preferably thin walled closure cap 17 may elastically deform the closure cap. The snap fit connector 12c on the end cap 12 comprises a third sloped surface 12i that is oriented in the circumferential direction (Figures 5a, 9a). The third sloped surface 12i may be on the same snap fit connector 12c having the first sloped surface 12d that is oriented in the axial direction as shown in Figure 5a. Alternatively the third sloped surface 12i is on a separate snap fit connector 12c. The third sloped surface 12i and the first sloped surface 12d are essentially oriented perpendicular to another. The snap fit connector 17b on the closure cap comprises a fourth surface 17f oriented in the circumferential direction and complimentary to the third sloped surface 12i on the snap fit connector 12c on the end cap 12 (Figure 9a). Rotation of the closure cap 17 with respect to the housing brings the third and fourth sloped surfaces 12i, 17f in engagement such that the relative movement between the sloped surfaces provides for a force in the radial direction thereby elastically deforming the closure cap 17 (Figure 9c). Once the closure cap has been fully rotated, optionally until the protrusion 17a of the closure cap abuts the radial stop 12f on the end cap, then the snap fit connectors 12c, 17b are disconnected (Figure 9d) and the closure cap 17 relaxes back to its original shape. The closure cap 17 may be axially removed from the injection device as this axial movement is allowed by the key/groove interaction 27f, 17e.

The stiffness of the snap fit connector 12c on the end cap 12 is greater than the stiffness of the closure cap 17 and/or the stiffness of the snap fit connector 17b on the closure cap. The stiffness may be tuned by the geometrical dimension, for example using a thin walled closure cap 17 with respect to snap fit connectors on the end cap having a greater thickness for example at least twice, preferably triple than the thickness of the closure cap. Alternatively, the snap fit connector 12c on the end cap is made from a material having a greater stiffness, for example by using a technical material such as a glass fibre reinforced plastic. The closure cap may be made from a material having a relatively low stiffness for example (expressed in the young's modulus) below 3 GPa, preferably below 2 GPa. The closure cap may be made for example from polypropylene, polyethylene, polycarbonate, ABS, polystyrene, a polyester such as polyethyleneterephthalate, a cycloolefinic polymer (COC or COP) or a polyamide. The polymers used may be based on recycled plastics or plastics made from non-fossil raw materials, alternatively biodegradable polymers are used. The closure cap may also be made from a blend of polymers such as polycarbonate/ABS or PPO/Polystyrene. Preferably, the closure cap 17 is thin walled and/ or made from a material having a relatively low stiffness as this saves material and costs.

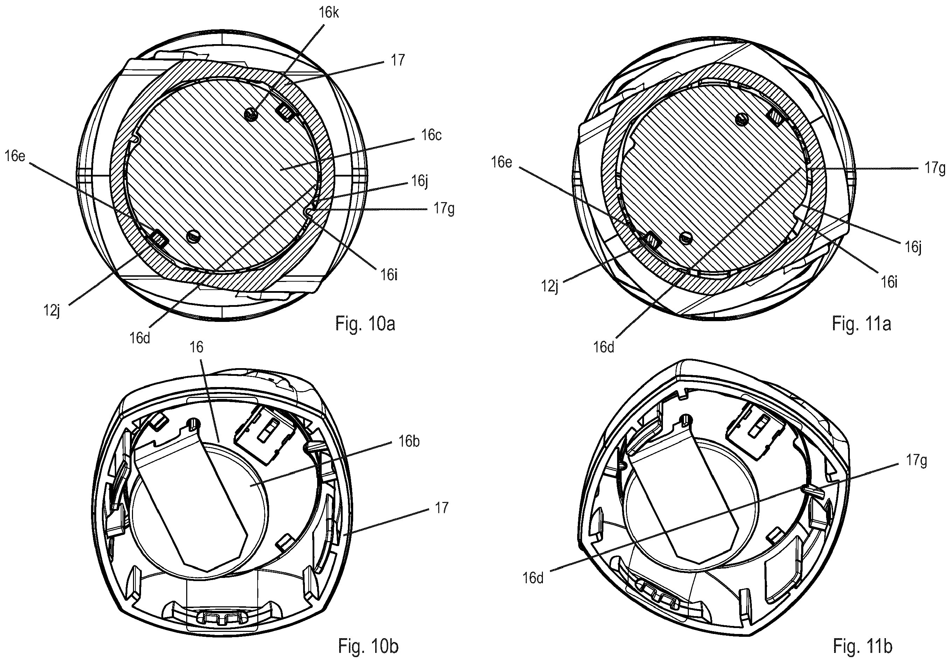

The fixation of the electronic module 16, preferably the fixation of the printed circuit board 16c is shown in Figures 10 to 13. The injection device is preferably fully assembled meaning that the closure cap 17 including the electronic module 16 is attached to the housing (2). Preferably, the manufacturer of the device has attached the electronic module to the injection device. Optionally, the injection pen closed by the end cap 12 is delivered as a kit of parts together with the closure cap 17 and the electronic module 16. The user or health care professional attaches the closure cap 17 with the electronic module 16 to the housing 2 or end cap 12 just prior use thereby obtaining the fully assembled injection device.

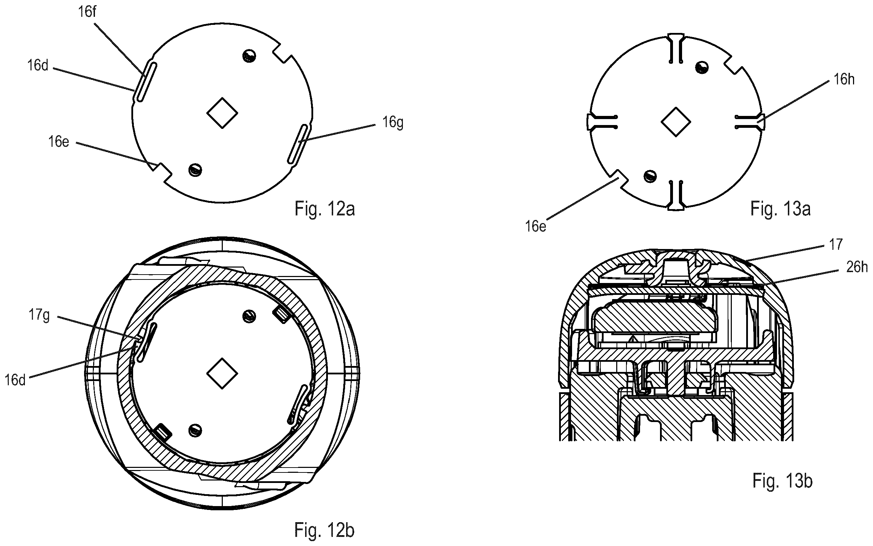

The PCB may be releasable connected to the closure cap once the closure cap 17 can be rotated and the connection between the closure cap and the housing has been released. The PCB 16c may be fixated to the closure cap 17 simultaneous with the release of the closure cap from the end cap, or the PCB 16c is fixated after the release of the closure cap and preferably using the same rotational movement. The PCB 16c is preferably rotationally fixated to the end cap 12 by cut outs 16e engaging protrusions 12j on the end cap 12 that extend in the proximal direction such that the PCB 16c cannot co-rotate with the closure cap 17 (Figure 10a). The engagement 12j, 16e allows for relative axial movement between the PCB 16c and the end cap 12 (Figure 10a). The closure cap 17 comprises a first connector 17g, preferably a protrusion that extends radially inward which fits into a cut out 16i in the PCB 16c before rotation of the closure cap 17. The PCB may have a second connector or connection means that is angular displaced from the cut out 16i. The cut out 16i may have a sloped section 16j. Rotation of the closure cap 17 in the counterclockwise direction rotates the closure cap 17 relative to the PCB 16c. The first connector or protrusion 17g is rotated relative to the PCB and optionally may be first compressed by the sloped section 16j on the PCB. The first connector 17g may finally engage the second connector 16d on the PCB 16c (Figure 11a). In this example, the second connector 16c represents the normal edge of the PCB without any modifications. Alternatively, the second connector 16c may be formed by another cut-out in the edge of the PCB. During rotation of the closure cap, the first connector 17g is elastically and/or plastically deformed to establish a connection between the closure cap 17 and the PCB 16c. The PCB is based on a rigid board and preferably does not deform during rotation of the closure cap 17. The closure cap 17 may be removed from the injection device together with the electronic module 16 and used for recycling purposes (Figure 11b). The PCB 16c may have additional holes 16k (also called release means) that may be used during recycling for rotating back (in the clockwise direction) the PCB 16c and releasing the PCB from the closure cap 17. Alternatively, the cut outs 16e may be used for releasing the PCB 16c from the closure cap 17. Alternatively, the elastic deformation is provided by the PCB (Figures 12 and 13). The PCB 16c may have slots 16g leaving thin sections 16f of the PCB such that radial forces may elastically deform the PCB 16c during rotation of the closure cap 17 (Figure 12b) thereby fixating the PCB to the closure cap. Alternatively, the PCB comprises arms 16 that protrude from the PCB 16c and that can flex such that the end of the arms may engage an internal surface of the closure cap (Figures 13a, 13b). After the closure cap 17 has been removed together with the PCB 16c from the injection device, the PCB may be released again from the closure cap either by elastically deforming (a part of) the PCB and/or elastically deforming (a part of) the closure cap.

The closure cap 17 may have an additional holding element or securing element 28 which sandwiches the electronic module 16, preferably the PCB 16c between the closure cap 17 and the housing 2 or the end cap 12 (Figures 4a, 4b). The securing element 28 may be an elastomeric member or a flexible arm or a combination thereof providing an interference fit for the PCB without deforming or stressing the PCB. The securing element presented in Figures 4a, 4b is an elastomeric element fitted into the dome of the closure cap and having a rim or wings extending distally that can flex and abut the proximal surface of the PCB. Before use, the PCB is fixated to the end cap 12 or housing 2 and is not loose within the space formed by the closure cap 17. The absence of mechanical stresses improves the reliability of the electronic module. After expelling medicament, the second releasable connection is formed between the closure cap 17 and the PCB and the connection may stress the PCB which may not disadvantageous as the electronic module has already been activated and used. The second releasable connection is formed for recycling purposes whereas the interference fit provided by the securing element before expelling medicament ensures that the module cannot move or vibrate and improves the impact resistance without jeopardizing the reliability of the PCB and its components.

### PART ANNOTATION

| | | | |
|---|---|---|---|
| 1 | Syringe holder | 12f | Radial stop |
| 1a | Arms | 12g | Guide slot |
| 2 | Housing | 12i | Third sloped surface |
| 3 | Needle cover sleeve | 12j | Protrusion |
| 3a | Proximal end | 13 | Wall |
| 3b | Recess | 14 | Needle shield remover |
| 4 | Pen cap | 15 | Switching sleeve |
| 5 | Mechanic holder | 15a | Distal end |
| 5c | Holding spring | 15b | Rim |
| 6 | Holding element | 15c | Cut out |
| 6a | Central pin, guide pin | 16 | Electronic module |
| 6b | Arms | 16a | Sensor/switch |
| 6c | Protrusion | 16b | Battery |
| 6d | Proximal end | 16c | PCB |
| 7 | Piston rod | 16d | Second connector |
| 7a | Recess | 16e | Cut out |
| 7b | Recess | 16f | Thin section |
| 8 | Locking sleeve | 16g | Slot |
| 8a | Flexible arm | 16h | Arm |
| 9 | Injection spring | 16i | Cut out |
| 9a | Distal end | 16j | Sloped section |
| 9b | Proximal end | 16k | Hole / passage |
| 10 | Needle cover sleeve spring | 17 | Closure cap |
| 10a | Distal end | 17a | Protrusion, guide pin |
| 10b | Proximal end | 17b | Snap fit connector |
| 11 | Signaling element | 17c | Second sloped surface |
| 11a | Protrusion | 17d | Second stopping surface |
| 11b | Arms | 17e | Key |
| 11c | Protrusion | 17f | Fourth sloped surface |
| 12 | End cap | 18 | Pivoting element |
| 12a | Radial stop | 19 | Bung |
| 12b | Step | 20 | Auto injector |
| 12c | Snap fit connector | 21 | Prefilled syringe |
| 12d | First sloped surface | 21a | Proximal end |
| 12e | First stopping surface | 22 | Needle shield |
| 23 | Hollow needle | 27c | Arms, hooks |
| 24 | LED | 27d | Fourth surface |
| 25 | Passage end cap | 27e | Gap |
| 26 | Securing element | 27f | Groove |
| 26a | Flexible arm | 27g | Centre portion |
| 26b | First sloped surface | 27h | Wings |
| 26c | Second sloped surface | 28 | Holding means |
| 26d | Crest | L | Longitudinal axis |
| 27 | Locking element, separation bolt | 8,15 | Switching module |
| 27a | Protrusion | | |
| 27b | Third sloped surface | | |

## Claims

1. An injection device for delivery of a fluid medicament comprising:
a housing (2) defining a longitudinal axis and enclosing an injection mechanism,
an end cap (12) fixated to the housing (2) delimiting the injection mechanism in a proximal direction,
a closure cap (17) releasably connected to the end cap thereby closing the injection device,
a releasable snap-fit connection between the end cap (17) and the closure cap (12),
**characterized in that** the releasable snap-fit connection is established before medicament delivery by relative axial movement along the longitudinal axis between the closure cap (17) and the housing (2) whereby a wall of the closure cap is elastically deformed, and the releasable snap-fit connection is configured to be released after medicament delivery by relative rotational movement around the longitudinal axis between the closure cap (17) and the housing (2) whereby the wall of the closure cap is elastically deformed.

2. The injection device according to claim 1 wherein the releasable snap-fit connection comprises a snap fit connector (12c) on the end cap (12) comprising a first sloped surface (12d) and a snap fit connector (17b) on the closure cap (17) comprising a second sloped surface (17c) whereby the first and the second sloped surfaces (12d, 17c) are configured to be brought into a sliding engagement during the relative axial movement along the longitudinal axis, and wherein the wall of the closure cap (17) is elastically deformed in a direction perpendicular to the longitudinal axis.

3. The injection device according to claim 2 wherein the snap-fit connector (12c) on the end cap (12) comprises a first stopping surface (12e) and the snap fit connector (17b) on the closure cap (17) comprises a second stopping surface (17d) adapted to engage the first stopping surface (12e) when the connection has been established thereby preventing the release of the releasable connection by relative axial movement between the closure cap (17) and the end cap (12) or housing (2).

4. The injection device according to claim 3 wherein the snap-fit connector (12c) on the end cap (12) comprises a third sloped surface (12i) oriented in the circumferential direction and the snap fit connector (17b) on the closure cap (17) comprises a complementary fourth sloped surface (17f), whereby the third and the fourth sloped surfaces (12i, 17f) are configured to be brought into a sliding engagement by relative rotational movement around the longitudinal axis between the closure cap (17) and the housing (2), and wherein the wall of the closure cap (17) is elastically deformed in a direction perpendicular to the axis.

5. The injection device according to claim 4 wherein the first and the third sloped surface (12d, 12i) on the end cap (12) are essentially oriented perpendicular to each other and the second and the fourth sloped surface (17c, 17f) on the closure cap (17) are essentially oriented perpendicular to each other.

6. The injection device according to any of the previous claims wherein the end cap (12) and/or the snap fit connector (12c) on the end cap has a stiffness that is greater than the stiffness of the wall of the closure cap (17).

7. The injection device according any of the previous claims wherein the thickness of the wall for the closure cap (17) is below 1 mm.

8. The injection device according to any of the previous claims wherein relative rotation between the closure cap (17) and the housing (2) before medicament delivery is prevented by a separation bolt (27) operatively coupled between the end cap (12) or housing (2) and the closure cap (17).

9. The injection device according to claim 8 wherein the separation bolt (27) is rotationally locked to the end cap (12) or housing (2) and rotationally locked to the closure cap (17) by at least two form fit engagements and wherein the separation bolt (27) is unlocked from the end cap (12) or housing (2) by the injection mechanism.

10. The injection device according to claim 9 wherein the injection mechanism is configured to axially move the separation bolt (27) to unlock the form fit engagement between the separation bolt (27) and the end cap (12) or housing (2).

11. The injection device according to claim 10 wherein the injection mechanism comprises a holding element (6) or a signaling element (11) configured to be moved in the proximal direction by an injection spring (9) upon medicament delivery and whereby the holding element (6) or the signaling element (11) is configured to move the separation bolt (27).

12. The injection device according to claim 11 wherein the injection spring (9) is kept in a compressed state by the holding element (6) before medicament delivery and configured to advance a plunger rod (7) for medicament delivery when the holding element (6) is released.

13. The injection device according to any of the previous claims wherein the closure cap (17) comprises a guide pin (17a) engaging a guide slot (12g) on the end-cap (12) or housing (2) thereby limiting the maximum rotation of the closure cap (17) with respect to the end cap (12) or housing (2).

14. The injection device according to any of the previous claims wherein the closure cap (17) defines a space between the closure cap (17) and the end cap (12) and wherein an electronic module (16) including a printed circuit board (16c) and a battery (16b) is located within the space.

15. The injection device according to claim 14 wherein the electronic module (16) is fixated to the closure cap (17) by relative rotation between the closure cap (17) and the end cap (12) or housing (2).
